(19) Europäisches Patentamt
European Patent Office
Office européen des brevets

(11) EP 0 884 048 B1

(12) **EUROPÄISCHE PATENTSCHRIFT**

(45) Veröffentlichungstag und Bekanntmachung des Hinweises auf die Patenterteilung:
**09.04.2003 Patentblatt 2003/15**

(51) Int Cl.[7]: **A61K 7/50**, A61K 7/48

(21) Anmeldenummer: **98109291.9**

(22) Anmeldetag: **22.05.1998**

(54) **Kosmetische und dermatologische Emulsionen, enthaltend Alkylglucoside und erhöhte Elektrolytkonzentrationen**

Cosmetic and dermatological emulsions containing alkylglucosides and increased electrolyte concentrations

Emulsions cosmétiques et dermatologiques contenant des alkylglucosides et des concentrations élevées d'électrolytes

(84) Benannte Vertragsstaaten:
**AT BE CH DE ES FR GB IT LI NL SE**

(30) Priorität: **06.06.1997 DE 19723733**

(43) Veröffentlichungstag der Anmeldung:
**16.12.1998 Patentblatt 1998/51**

(73) Patentinhaber:
- **Beiersdorf Aktiengesellschaft**
  **20245 Hamburg (DE)**
- **Goldschmidt AG**
  **45127 Essen (DE)**

(72) Erfinder:
- **Kröpke, Rainer**
  **22527 Hamburg (DE)**
- **Lührs, Anja**
  **22399 Hamburg (DE)**
- **Müller, Anja**
  **23843 Rümpel (DE)**
- **Nielsen, Jens**
  **24558 Henstedt-Ulzburg (DE)**
- **Bungard, Andrea, Dr.**
  **45136 Essen (DE)**
- **Grüning, Burghard, Dr.**
  **45134 Essen (DE)**
- **Jenni, Klaus, Dr.**
  **58454 Witten (DE)**

(56) Entgegenhaltungen:
**EP-A- 0 384 983**
**EP-A- 0 388 810**
**EP-A- 0 408 965**
**EP-A- 0 750 034**
**DE-A- 4 302 315**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 884 048 B1

Printed by Jouve, 75001 PARIS (FR)

## Beschreibung

Die vorliegende Erfindung betrifft kosmetische und dermatologische Emulsionen, insbesondere hautpflegende kosmetische und dermatologische Emulsionen. In einer vorteilhaften Ausführungsform betrifft die vorliegende Erfindung eine Anwendung, welche es erlaubt, die Stabilität von Emulsionen, insbesondere von O/W-Emulsionen zu steigern.

Die äußerste Schicht der Epidermis, das Stratum comeum (Hornschicht), ist als wichtige Barriereschicht von besonderer Bedeutung u.a. für den Schutz vor Umwelteinflüssen und Austrocknung. Die Hornschicht wird im Kontakt mit der Umwelt ständig abgenutzt und muß deshalb ununterbrochen erneuert werden.

Ein heute in der Fachwelt weitverbreitetes Hautmodell faßt das Stratum corneum als Zwei-Komponenten-System, ähnlich einer Ziegelsteinmauer (Ziegelstein-Mörtel-Modell), auf. In diesem Modell entsprechen die Korneozyten (Hornzellen) den Ziegelsteinen, die kompliziert zusammengesetzte Lipidmembran in den Interzellularräumen entspricht dem Mörtel.

Außer ihrer Barrierewirkung gegen externe chemische und physikalische Einflüsse tragen die epidermalen Lipide auch zum Zusammenhalt der Hornschicht bei und haben Einfluß auf die Hautglätte. Im Gegensatz zu den Talgdrüsenlipiden, die keinen geschlossenen Film auf der Haut ausbilden, sind die epidermalen Lipide über die gesamte Homschicht verteitt.

Das äußerst komplexe Zusammenwirken der feuchtigkeitsbindenden Substanzen und der Lipide der oberen Hautschichten ist für die Regulation der Hautfeuchte sehr wichtig.

Daher enthalten Kosmetika in der Regel, neben ausgewogenen Lipidabmischungen und Wasser, wasserbindende Substanzen.

Neben der chemischen Zusammensetzung ist jedoch auch das physikalische Verhalten dieser Substanzen von Bedeutung. Daher ist die Entwicklung von sehr gut bioverträglichen Emulgatoren bzw. Tensiden mit flüssigkristallinen Eigenschaften wünschenswert. Damit formulierte Produkte unterstützen die flüssigkristalline Organisation der Interzellularlipide des Stratum Comeums und verbessern so die Barriereeigenschaften der Hornschicht. Besonders vorteilhaft ist es, wenn deren Molekülbestandteile aus natürlicherweise in der Epidermis vorkommenden Substanzen bestehen.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es femer, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Übliche kosmetische Darreichungsformen sind Emulsionen, also metastabile Zwei- oder Mehrphasensysteme bei welchen die einzelnen Phasen im flüssigen Zustande vorliegen. Die gängigsten Emulsionen sind O/W- und W/O-Emulsionen. Seltenere Darreichungsformen sind multiple Emulsionen, also solche, welche in den Tröpfchen der dispergierten (oder diskontinuierlichen) Phase ihrerseits Tröpfchen einer weiteren dispergierten Phase enthalten, z.B. W/O/W-Emulsionen und O/W/O-Emulsionen.

Um die Metastabilität von Emulsionen gewährleisten zu können, sind in der Regel grenzflächenaktive Substanzen, also Emulgatoren, nötig.

An sich ist die Verwendung der üblichen kosmetischen Emulgatoren völlig unbedenklich. Dennoch können Emulgatoren, wie letztlich jede chemische Substanz, im Einzelfalle allergische oder auf Überempfindlichkeit des Anwenders beruhende Reaktionen hervorrufen.

So ist bekannt, daß bei manchen besonders empfindlichen Personen bestimmte Lichtdermatosen durch gewisse Emulgatoren und gleichzeitige Einwirkung von Sonnenlicht ausgelöst werden.

Es ist möglich, emulgatorfreie Zubereitungen herzustellen, welche beispielsweise in einer wäßrigen Phase dispergierte Öltröpfchen, ähnlich einer O/W-Emulsion, aufweisen. Voraussetzung dafür kann sein, daß die kontinuierliche wäßrige Phase ein die dispergierte Phase stabilisierendes Gelgerüst aufweist und andere Umstände mehr. Solche Systeme werden gelegentlich Hydrodispersionen oder Oleodispersionen genannt, je nachdem, welches die disperse und welches die kontinuierliche Phase darstellt.

Es ist für die kosmetische Galenik aber weder nötig noch möglich, auf Emulgatoren ganz zu verzichten, zumal

eine gewisse Auswahl an besonders milden Emulgatoren existiert. Allerdings besteht ein Mangel des Standes der Technik an einer befriedigend großen Vielfalt solcher Emulgatoren, welche dann auch das Anwendungsspektrum entsprechend milder und hautverträglicher kosmetischer Zubereitungen deutlich verbreitem würde.

So war eine Aufgabe der vorliegenden Erfindung, kosmetische bzw. dermatologische Zubereitungen mit hervorragenden hautpflegenden Eigenschaften zur Verfügung zu stellen.

Ein Nachteil insbesondere von O/W-Emulsionen ist oft deren mangelnde Stabilität gegenüber höheren Elektrolytkonzentrationen, was sich in Phasentrennung äußert. Dies kann zwar auch bei W/O-EMulsionen gelegentlich zu Problemen führen, tritt dort aber bei weitem nicht so in den Vordergrund wie bei O/W-Systemen. Zwar läßt sich diesen oft durch geeignete Wahl des Emulgatorsystems in gewissem Maße Abhilfe schaffen, es treten dann aber ebensooft andere Nachteile auf.

Es ist andererseits oft wünschenswert, bestimmte Elektrolyte einzusetzen, um deren sonstige physikalische, chemische bzw. physiologische Eigenschaften nutzen zu können.

Üblicherweise werden die Konzentrationen aller Bestandteile einer kosmetischen oder dermatologischen Zubereitungen in solchen Einheiten wie Gewichts-%, Mol-% und dergleichen angegeben. Aufgrund ihrer mehr oder minder stark ausgeprägten Dissoziation in Kationen und Anionen, oftmals in mehreren Dissoziationsstufen, erscheint es zweckmäßiger für die Schilderung der vorliegenden Erfindung und ihres technischen Hintergrundes, von der Ionenstärke eines gegebenen Elektrolytes in seiner Lösung auszugehen.

Die Ionenstärke *I* einer Elektrolytlösung ist definiert als

$$I = \frac{1}{2} \sum_i c_i z_i^2$$

wobei $c_i$ die Konzentrationen der einzelnen Ionensorten (in mol/l) und $z_i$ deren Ladungszahlen darstellen. Die physikalische Einheit der Ionenstärke ist die einer Konzentration (mol/l).

Eine 1-%ige ( = 0,17-molare) Kochsalzlösung hat beispielsweise eine Ionenstärke *I* = 0,17.

Eine weitere Aufgabe der vorliegenden Erfindung war es also, Lösungswege zu kosmetischen oder dermatologischen Emulsionen, insbesondere O/W-Emulsionen, aufzudekken, welche gegenüber erhöhten Elektrolytkonzentrationen - bzw. erhöhten Ionenstärken - stabil sind. Femer war eine Aufgabe der vorliegenden Erfindung, Zubereitungen zur Verfügung zu

stellen, welche den Zustand der Haut deutlich verbessern, insbesondere die Hautrauhigkeit vermindern.

Es hat sich überraschend gezeigt, und darin liegt die Lösung dieser Aufgaben, daß kosmetische und dermatologische Emulsionen mit mindestens einer wäßrigen Phase, enthaltend

(a) eine wirksame Menge einer oder mehrerer grenzflächenaktiven Substanzen, gewählt aus der Gruppe der Alkylglucoside, welche sich durch die Strukturformel

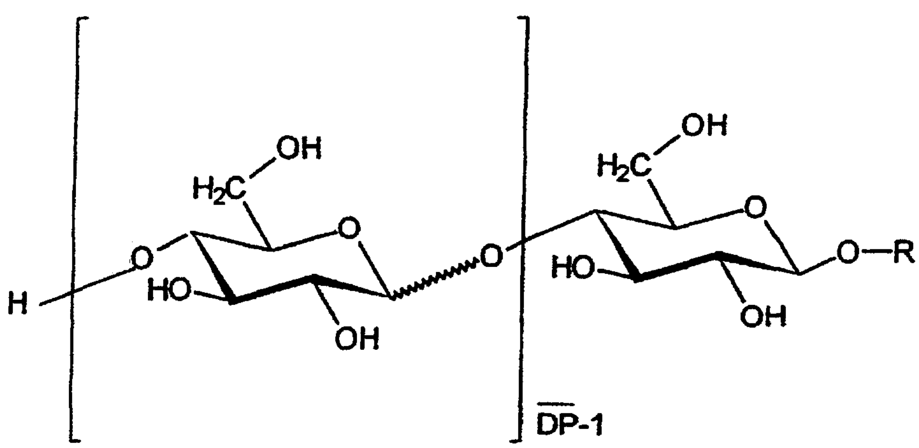

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet,

(b) wobei mindestens eine der wäßrigen Phasen einen oder mehrere Elektrolyte gelöst enthält, wobei die Ionenstärke der wäßrigen Phasen, in der der oder die Elektrolyte gelöst vorliegen, mindestens 0,075 mol/l beträgt,

den Nachteilen des Standes der Technik abhelfen.

Es war für den Fachmann daher nicht vorauszusehen gewesen, daß die erfindungsgemäßen Zubereitungen

- besser als feuchtigkeitsspendende Zubereitungen wirken,
- besser die Hautglättung fördern,
- sich durch besser Pflegewirkung auszeichen,
- besser als Vehikel für kosmetische und medizinisch-dermatologische Wirkstoffe dienen
- höhere Stabilität gegenüber Zerfall in Öl- und Wasserphasen aufweisen und
- sich durch bessere Bioverträglichkeit auszeichnen würden

als die Zubereitungen des Standes der Technik.

Zwar beschreibt die EP-A-0 629 396 stabile kosmetische Emulsionen, welche sich durch einen Gehalt an Alkylglucosiden auszeichnen. Gemäß Anspruch 6 a.a.O. können die Zubereitungen wohl auch Salze enthalten. Die aufgrund der Gewichtskonzentrationen in den Beispielen konkret offenbarten Ionenstärken der dort angegebenen Elektrolyte sind mit 0,046 mol/l (für Natriumhydroxid in Beispiel 1), 0,023 mol/l (für Natriumhydroxid in Beispiel 2) bzw. 0,03 mol/l für $Na_2H_2$EDTA in Beispiel 5) viel zu niedrig, als daß die erfindungsgemäße Lehre auch nur nahegelegt würde.

In EP-A-0 629 396, Beispiel 6, wird noch eine weitere stabile kosmetische Emulsion mit einem Elektrolyten der Ionenstärke 0,023 mol/l (für Natriumhydroxid) angegeben, wobei dieses Beispiel 6 sich auf Zubereitungen, ebenfalls mit einem Gehalt an Alkylglucosiden, allerdings gemäß der Schrift WO 92/06778, bezieht. Letztere Schrift schweigt sich zur Verwendung von Elektrolyten gänzlich aus. Der Stand der Technik konnte infolgedessen nicht den Weg zur vorliegenden Erfindung bereiten.

Erfindungsgemäß ist femer auch die nichttherapeutische Verwendung von kosmetischen und dermatologischen Emulsionen, insbesondere O/W-Emulsionen mit mindestens einer wäßrigen Phase, enthaltend

(a) eine wirksame Menge einer oder mehrerer grenzflächenaktiven Substanzen, gewählt aus der Gruppe der Alkylglucoside, welche sich durch die Strukturformel

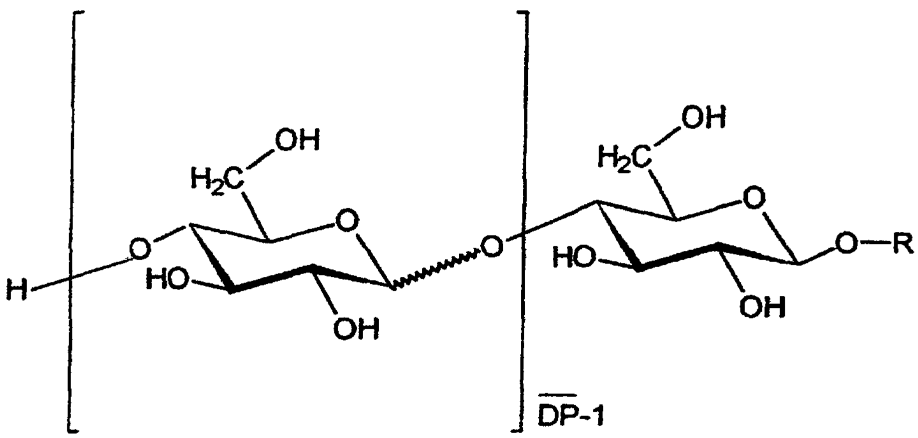

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet,

(b) wobei mindestens eine der wäßrigen Phasen einen oder mehrere Elektrolyte gelöst enthält, wobei die Ionenstärke der wäßrigen Phasen, in der der oder die Elektrolyte gelöst vorliegen, mindestens 0,075 mol/l beträgt,

zur Pflege der Haut.

Erfindungsgemäß ist weiterhin auch die Verwendung einer oder mehrerer grenzflächenaktiver Substanzen, gewählt aus der Gruppe der Alkylglucoside, welche sich durch die Strukturformel

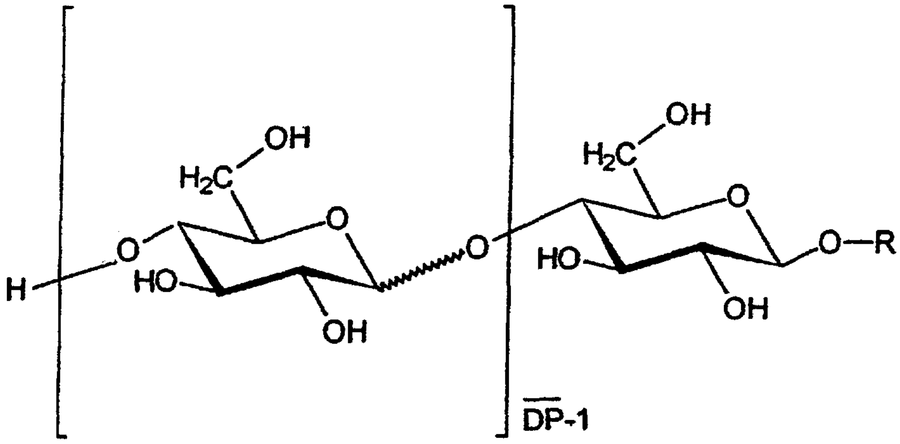

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet, zum Erzielen oder Erhöhen der Stabilität von Emulsionen gegenüber der Gegenwart von Elektrolyten, insbesondere zum Erzielen oder Erhöhen der Stabilität von O/W-Emulsionen gegenüber der Gegenwart von Elektrolyten.

Erfindungsgemäß ist insbesondere die Verwendung einer oder mehrerer grenzflächenaktiver Substanzen, gewählt aus der Gruppe der Alkylglucoside, welche sich durch die Strukturformel

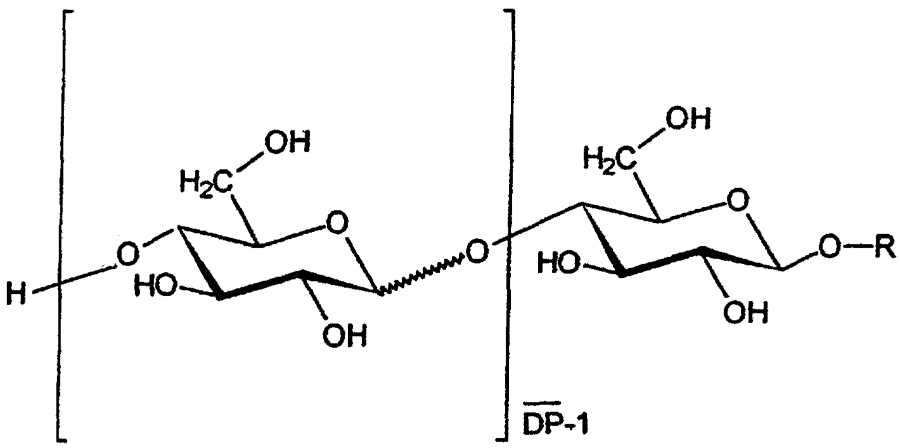

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet, zum Erzielen oder Erhöhen der Stabilität von Emulsionen gegenüber der Gegenwart von Elektrolyten, insbesondere zum Erzielen oder Erhöhen der Stabilität von O/W-Emulsionen gegenüber der Gegenwart von Elektrolyten, wobei die Emulsionen in mindestens einer der wäßrigen Phasen einen oder mehrere Elektrolyte gelöst enthält, und die Ionenstärke der wäßrigen Phasen, in der der oder die Elektrolyte gelöst vorliegen, mindestens 0,075 mol/l beträgt.

Der Wert $\overline{DP}$ repräsentiert den Glucosidierungsgrad der erfindungsgemäß verwendeten Alkylglucosiden und ist definiert als

$$\overline{DP} = \frac{p_1}{100} \cdot 1 + \frac{p_2}{100} \cdot 2 + \frac{p_3}{100} \cdot 3 + \ldots = \sum_i \frac{p_i}{100} \cdot i$$

Dabei stellen $p_1$, $p_2$, $p_3$ ... bzw, $p_i$ den Anteil der einfach, zweifach dreifach ... i-fach glucosylierten Produkte in Gewichtsprozenten dar. Erfindungsgemäß vorteilhaft werden Produkte mit Glucosylierungsgraden von 1 - 2, insbesondere vorteilhaft von 1,1 bis 1,5, ganz besonders vorteilhaft von ungefähr 1,3 gewählt.

Der Wert DP trägt dem Umstande Rechnung, daß Alkylglucoside herstellungsbedingt in der Regel Gemische aus Mono- und Oligoglucosiden darstellen. Erfindungsgemäß vorteilhaft ist ein relativ hoher Gehalt an Monoglucosiden, typischerweise in der Größenordnung von 40 - 70 Gew.-%.

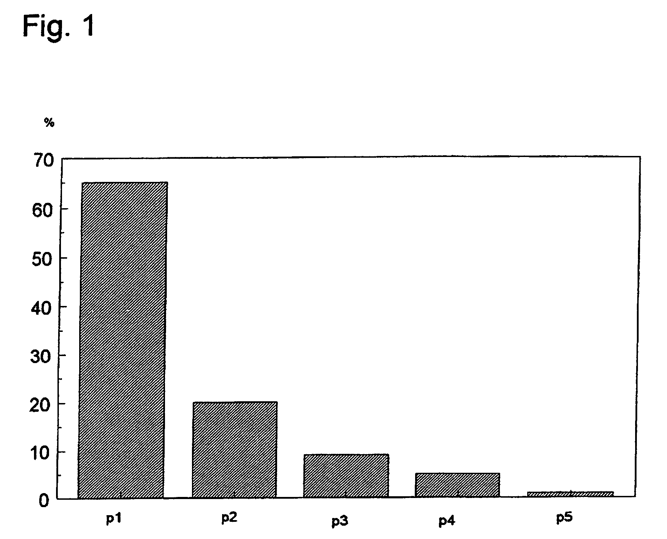

Eine typische Oligomerenverteilung für einen Glucosylierungsgrad von etwa 1,3 ist in Fig. 1 dargestellt.

Vorteilhaft wird R gewählt aus der Gruppe der unverzweigten Alkylreste, wobei der Myristylrest, der Cetylrest, der Stearylrest und der Eicosylrest bevorzugt werden.

Erfindungsgemäß eingesetzte Alkylglucoside sind erhältlich durch Verfahren, wie sie beispielsweise in der DE-OS 40 40 655 und anderen Schriften beschrieben werden. Sie sind im Handel von verschiedenen Herstellern erhältlich.

Beispielsweise vorteilhaft ist es, Gemische aus Stearylglucosid und Cetylglucosid zu verwenden. Solche Mischungen sind im Handel beispielsweise unter der Warenbezeichnung Tego® Care SG 90 von der Gesellschaft Th. Goldschmidt KG erhältlich.

Die Gesamtmenge an einer oder mehreren erfindungsgemäß verwendeten grenzflächenaktiven Glucosederivaten in den fertigen kosmetischen oder dermatologischen Zubereitungen wird vorteilhaft aus dem Bereich von 0,1 - 25,0 Gew.-%, bevorzugt 0.5 - 15,0 Gew.-% gewählt, bezogen auf das Gesamtgewicht der Zubereitungen.

Erfindungsgemäß werden der oder die Elektrolyte vorteilhaft gewählt aus der Gruppe

(1) der Salze mit folgenden Anionen: Chloride, femer anorganische Oxo-Element-Anionen, von diesen insbesondere Sulfate, Carbonate, Phosphate, Borate und Aluminate. Auch auf organischen Anionen basierende Elektrolyte sind vorteilhaft, z.B. Lactate, Acetate, Benzoate, Propionate, Tartrate, Citrate, Aminosäuren und deren Salze und andere mehr. Vergleichbare Effekte sind auch durch Ethylendiamintetraessigsäure und deren Salze zu erzielen.

Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Besonders bevorzugt sind Kaliumchlorid, Kochsalz, Magnesiumsulfat, Zinksulfat und Mischungen daraus.

Erfindungsgemäß werden der oder die Elektrolyte ferner vorteilhaft gewählt aus der Gruppe

(2) Bestimmte wasserlösliche, zumeist als Alkalisalze vorliegende wasserlösliche UV-Fittersubstanzen, insbesondere solche, die an ihrem Molekülgerüst eine oder mehrere Sulfonsäuregruppen bzw. Sulfonatgruppen tragen:

Die 2-Phenylbenzimidazol-5-sulfonsäure und ihre Salze, beispielsweise das Natrium-, Kalium- oder ihr Triethanolammonium-Salz

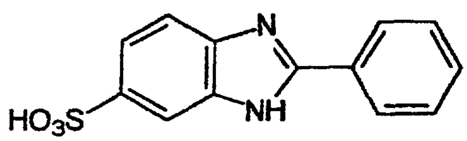

,

Sulfonsäure-Derivate von Benzophenonen, vorzugsweise 2-Hydroxy-4-methoxybenzophenon-5-sulfonsäure und ihre Salze, beispielsweise das entsprechende Natrium-, Kalium- oder Triethanolammonium-Salz:

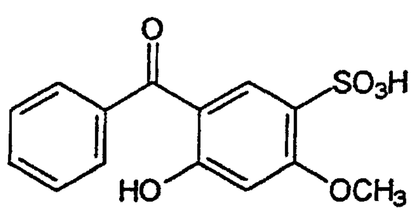

Sulfonsäure-Derivate des 3-Benzylidencamphers, wie z.B. 4-(2-Oxo-3-bomylidenmethyl)benzolsulfonsäure und ihre Salze, beispielsweise das entsprechende Natrium-, Kalium- oder Triethanolammonium-Salz:

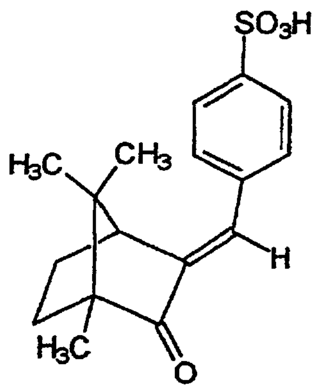

Die 2-Methyl-5-(2-oxo-3-bornylidenmethyl)benzolsulfonsäure und ihre Salze, beispielsweise das entsprechende Natrium-, Kalium- oder Triethanolammonium-Salz:

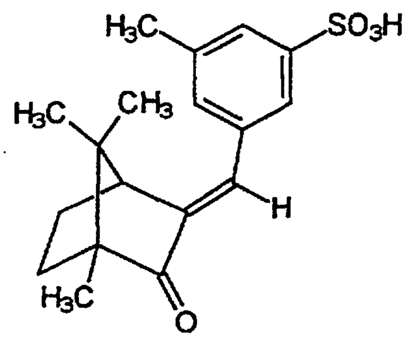

Das 1,4-di(2-oxo-10-Sulfo-3-bornylidenmethyl-)Benzol und dessen Salze (die entprehenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet:

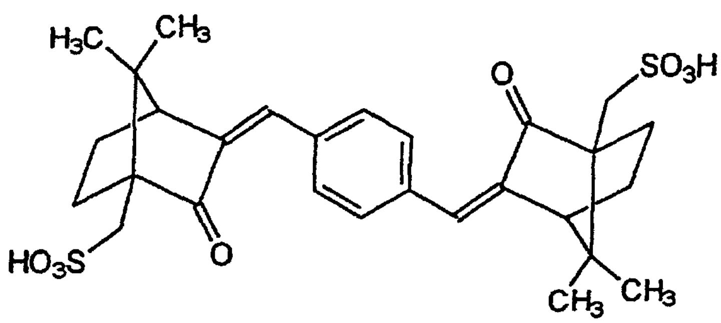

Erfindungsgemäß werden der oder die Elektrolyte weiterhin vorteilhaft gewählt aus der Gruppe

(3) der Aminosäuren und deren Salze bzw. deren Anionen. Aminosäuren sind Bestandteil des natürlichen Feuchtigkeitsfaktors (der sogenannte Natural Moisturizing Factor). Der Zusatz von Aminosäuren, insbesondere essentieller Aminosäuren, ist als vorteilhaft anzusehen, da über Hydratationsvorgänge Feuchtigkeit in der Haut gebunden werden kann.

Aminosäuren mit besonders vorteilhafter kosmetischer bzw. dermatologischer Wirkung sind Glycin, Alanin, Valin, Leucin, Isoleucin, Phenylalanin, Tyrosin, Prolin, Hydroxyprolin, Serin, Threonin, Cystein, Methionin, Tryptophan, Arginin.

Erfindungsgemäß werden der oder die Elektrolyte weiter vorteilhaft gewählt aus der Gruppe

(4) der kosmetisch und dermatologisch relevanten α-Hydroxycarbonsäuren, α-Ketocarbonsäuren und β-Hydroxycarbonsäuren und insbesondere deren Salze, wobei die Kationen vorteilhaft gewählt werden können aus der Gruppe Ammonium,-Alkylammonium,- Alkalimetall-, Erdalkalimetall,- Magnesium-, Eisen- bzw. Zinkionen.

α-Hydroxycarbonsäuren, welche kosmetisch oder dermatologisch relevant sind, folgen der allgemeinen Formel

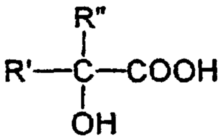

β-Hydroxycarbonsäuren, welche kosmetisch oder dermatologisch relevant sind, folgen der allgemeinen Formel

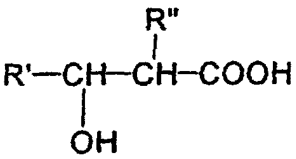

bzw.

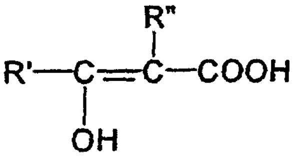

α-Ketocarbonsäuren, welche kosmetisch oder dermatologisch relevant sind, folgen der allgemeinen Formel

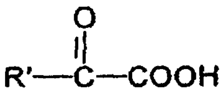

wobei jeweils R' und R" unabhängig voneinander gewählt werden aus der Gruppe

(a1) H-,
(a2) verzweigtes oder unverzweigtes $C_{1-25}$-Alkyl-,
(a3) mit einer oder mehreren Carboxylgruppen und/oder Hydroxygruppen und/oder Aldehydgruppen und/oder Oxogruppen (Ketogruppen) substituiertes verzweigtes oder unverzweigtes $C_{1-25}$-Alkyl-
(a4) Phenyl-,
(a5) mit einer oder mehreren Carboxylgruppen und/oder Hydroxygruppen und/oder verzweigten und/oder unverzweigten $C_{1-25}$-Alkylgruppen substituiertes Phenyl-,
oder wobei das α-Kohlenstoffatom und das β-Kohlenstoffatom der β-Hydroxycarbonsäure mit R' und R" zusammen eine
(a6) unsubstituierte Cycloalkylgruppe oder Arylgruppe mit 3 bis 7 Ringatomen oder eine
(a7) mit einer oder mehreren Carboxylgruppen und/oder Hydroxygruppen und/oder Oxogruppen (Ketogruppen) und/oder verzweigten und/oder unverzweigten $C_{1-25}$-Alkylgruppen substituierte Cycloalkylgruppe oder Arylgruppe mit 3 bis 7 Ringatomen
ausbildet und
wobei die α-Hydroxycarbonsäuren oder die β-Hydroxycarbonsäuren oder die α-Ketocarbonsäuren gegebenenfalls in Form ihrer physiologisch verträglichen Salze vorliegen können.

Es folgen vorteilhaft im Rahmen der vorliegenden Erfindung zu verwendende α-Hydroxycarbonsäuren, β-Hydroxycarbonsäuren und α-Ketocarbonsäuren, wobei diese auch stellvertretend für ihre Salze bzw. Anionen aufgeführt werden:

Die Salicylsäure (auch 2-Hydroxybenzoësäure, Spirsäure), welche durch die Struktur

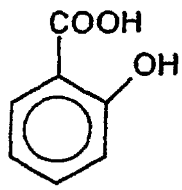

gekennzeichnet ist. Bekanntermaßen wirkt Salicylsäure antibakteriell und keratolytisch und ist Bestandteil mancher kosmetischen oder pharmazeutischen Zubereitungen.

Die den erfindungsgemäß verwendeten α-Hydroxycarbonsäuren werden vorteilhaft gewählt aus folgenden Substanzklassen:

(a2) α-Hydroxyfettsäuren, wobei diese wiederum besonders vorteilhaft aus der Gruppe der $C_{10-18}$-Alkylcarbonsäuren gewählt werden,
(a3) α-Hydroxyzuckersäuren, aliphatische α-Hydroxyfruchtsäuren.
(a4) unsubstituierte aromatische α-Hydroxycarbonsäuren (z.B. Mandelsäure) bzw.
(a5) substituierte aromatische α-Hydroxycarbonsäuren.

Die unter Punkt (a2) fallenden α-Hydroxyfettsäuren werden besonders vorteilhaft gewählt aus der Gruppe

- α-Hydroxycarbonsäuren, gemäß der Formel

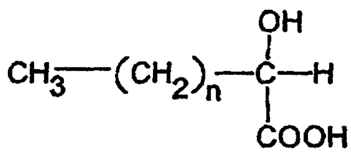

und/oder

- α-Hydroxy-isocarbonsäuren, gemäß der Formel

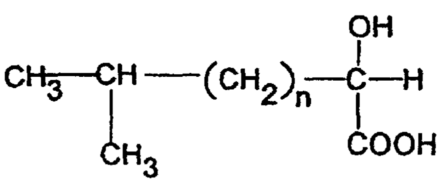

und/oder

- α-Hydroxy-anteisocarbonsäuren, gemäß der Formel

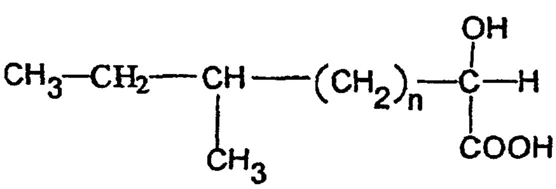

,

wobei n jeweils eine Zahl von 7 bis 31 darstellt.

Vorteilhaft ist weiter, Gemische solcher aliphatischen α-Hydroxycarbonsäuren, insbesondere in Form von Wollwachssäuregemischen zu verwenden, in welchen der Gehalt an α-Hydroxycarbonsäuren 20 - 30 Gew.-%, bezogen auf die Gesamtzusammensetzung beträgt.

Die unter Punkt (a3) fallenden α-Hydroxyzuckersäuren werden besonders vorteilhaft gewählt aus der Gruppe der

- Aldonsäuren, z.B. Gluconsäure, Galactonsäure
- Aldarsäuren, z.B. Glucarsäure, Galactarsäure (aber auch die Fruchtsäure Weinsäure, die ebenfalls unter die Definition der Aldarsäure fällt)
- Uronsäuren, z.B. Glucuronsäure, Galacturonsäure
- Glycerinsäure

Die unter Punkt (a3) fallenden aliphatischen α-Hydroxyfruchtsäuren werden besonders vorteilhaft gewählt aus der Gruppe Äpfelsäure, Milchsäure, Citronensäure, Weinsäure.

Äpfelsäure (Hydroxybernsteinsäure) ist durch folgende chemische Struktur gekennzeichnet:

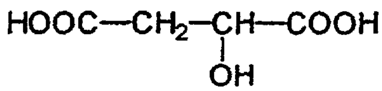

Milchsäure (2-Hydroxypropansäure) ist durch folgende chemische Struktur gekennzeichnet:

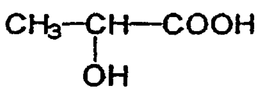

Citronensäure (2-Hydroxy-1,2,3-propantricarbonsäure) ist durch folgende chemische Struktur gekennzeichnet:

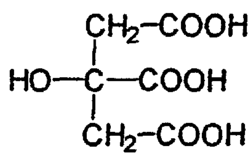

Bekanntermaßen wird Citronensäure zur Pufferung kosmetischer und/oder dermatologischer Zubereitungen, aber auch als Synergist für Antioxidantien in der Haut- und Haarkosmetik verwendet.

Weinsäure (Dihydroxybemsteinsäure) ist durch folgende chemische Struktur gekennzeichnet:

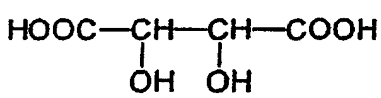

Bevorzugte α-Ketocarbonsäure ist die Brenztraubensäure (α-Oxopropansäure). Sie zeichnet sich durch folgende Struktur aus:

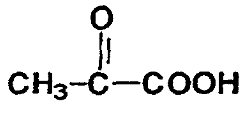

Die Höchstmenge der einzusetztenden Elektrolyte ist letztendlich abhängig von deren Löslichlichkeit in der wäßrigen Phase. Grundsetzlich setzt die erfindungsgemäße Lehre aber keine Höchstmengen als Schranke, da es gegebenenfalls ja sogar vorteilhaft sein mag, aus welchen Gründen auch immer, einen über die Löslichkeit eines Elektrolytes hinausgehende zusätzliche Menge dieses Elektrolytes, beispielsweise als ungelösten Festkörper, in eine kosmetische oder dermatologische Zubereitung einzuarbeiten.

Erfindungsgemäße kosmetische und dermatologische Zubereitungen enthalten vorteilhaft außerdem anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$), Zirkoniums ($ZrO_2$), Silici-

ums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Es ist besonders vorteilhaft im Sinne der vorliegenden Erfindung, wenngleich nicht zwingend, wenn die anorganischen Pigmente in hydrophober Form vorliegen, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Rektion gemäß

$$n\ TiO_2 + m\ (RO)_3\ Si\text{-}R' \rightarrow n\ TiO_2\ (\text{oberfl.})$$

erzeugt wird. n und m sind dabei nach Belieben einzusetzende stöchiometrische Parameter. R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen MT 100 T von der Firma TAYCA, femer M 160 von der Firma Kemira sowie T 805 von der Firma Degussa erhältlich.

Die erfindungsgemäßen kosmetischen und/oder dermatologischen Lichtschutzformulierungen können wie üblich zusammengesetzt sein und dem kosmetischen und/oder dermatologischen Lichtschutz, femer zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Camosin und deren Derivate (z.B. Anserin), Carotinoide, Carotine (z.B. $\alpha$-Carotin, $\beta$-Carotin, $\psi$-Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-Apalmitat) sowie Koniferylbenzoat des Benzoeharzes, Rutinsäure und deren Derivate, $\alpha$-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen

auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Femer kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkemöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkybenzoat und 2-Ethylhexylisostearat, Mischungen aus $C_{12-15}$-Alkybenzoat und Isotridecylisononanoat sowie Mischungen aus $C_{12-15}$-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind femer Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, femer Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid. Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die kosmetischen oder dermatologischen Lichtschutzzubereitungen enthalten vorteilhaft anorganische Pigmente, insbesondere Mikropigmente, z.B. in Mengen von 0,1 Gew.-% bis 30 Gew.-%, vorzugsweise in Mengen von 0,5 Gew.-% bis 10 Gew.-%, insbesondere aber 1 Gew.-% bis 6 Gew.-%, bezogen auf das Gesamtgewicht der Zubereitungen.

Es ist erfindungsgemäß vorteilhaft, außer den erfindungsgemäßen Kombinationen öllösliche UVA-Filter und/oder UVB-Filter in der Lipidphase und/oder wasserlösliche UVA-Filter und/oder UVB-Filter in der wäßrigen Phase einzusetzen.

Vorteilhaft können die erfindungsgemäßen Lichtschutzformulierungen weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die weiteren UVB-Filter können öllöslich oder wasserlöslich sein. Vorteilhafte öllösliche UVB-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;

Vorteilhafte wasserlösliche UVB-Filtersubstanzen sind bereits unter dem Begriff der erfindungsgemäß vorteilhaften Elektrolyte genannt worden, seien hier aber noch einmal auszugsweise genannt:

- Satze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und deren Salze;
- Suifonsäure-Derivate des 3-Benzylidencamphers, wie z.B. 4-(2-Oxo-3-bomylidenmethyl)benzolsulfonsäure,2-Methyl-5-(2-oxo-3-bornylidenmethyl)sulfonsäure und deren Salze.

Die Liste der genannten weiteren UVB-Filter, die in Kombination mit den erfindungsgemäßen Wirkstoffkombinationen verwendet werden können, soll selbstverständlich nicht limitierend sein.

Es kann auch von Vorteil sein, die erfindungsgemäßen Kombinationen mit weiteren UVA-Filtem zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Femer ist vorteilhaft, die erfindungsgemäßen Wirkstoffkombinationen mit weiteren UVAund/oder UVB-Filtern zu kombinieren.

Es ist auch besonders vorteilhaft, die erfindungsgemäßen Wirkstoffkombinationen mit Salicylsäurederivaten zu kombinieren, von denen einige Vertreter bekannt sind, welche ebenfalls UV-Strahlung absorbieren können. Zu gebräuchlichen UV-Filtern gehören

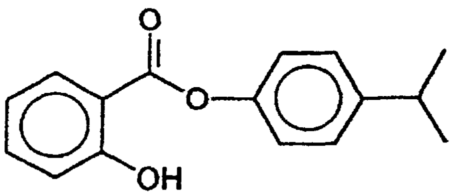

(4-Isopropylbenzylsalicylat),

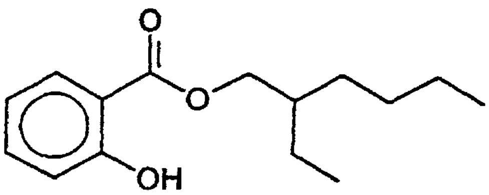

(2-Ethylhexylsalicylat, Octylsalicylat),

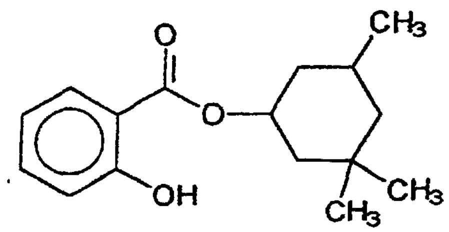

(Homomenthylsalicylat).

Die Herstellung der erfindungsgemäßen kosmetischen und/oder dermatologischen Zubereitungen erfolgt in der dem Fachmann üblichen Weise, zumeist dergestallt, daß eine Ölphase mit einem Gehalt an erfindungsgemäß verwendeten grenzflächenaktiven Glucosederivaten bei gleichmäßigem Rühren und gegebenenfalls unter Erwärmen suspendiert und gewünschtenfalls homogenisiert, gegebenenfalls mit weiteren Lipidkomponenten und gegebenenfalls mit einem oder mehreren weiteren Emulgatoren vereinigt, hernach die Ölphase mit der wäßrigen Phase, in welche gegebenenfalls ein Verdickungsmittel eingearbeitet worden ist, und welche vorzugsweise etwa die gleiche Temperatur besitzt wie die Ölphase, vermischt, gewünschtenfalls homogenisiert und auf Raumtemperatur abkühlen läßt. Nach Abkühlen auf Raumtemperatur kann, insbesondere, wenn noch flüchtige Bestandteile eingearbeitet werden sollen, nochmaliges Homogenisieren erfolgen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Beispiel 1**

| O/W-Lotion | |
|---|---|
| Glycerylstearat SE | 3.50 |
| Tego Care CG 90 | 1.80 |
| Glycerin | 3.00 |
| Cetearylalkohol | 0.50 |
| Octytdodecanol | 7.0 |
| Caprylylether | 8.0 |
| Eusolex 232 | 3.0 |
| NaOH (45%ig) | 1.0 |
| Cetearyl Isononanoat | 6.0 |
| Carbomer | 0.20 |

(fortgesetzt)

| O/W-Lotion | |
|---|---|
| Konservierungsmittel | q.s. |
| Parfum | q.s. |
| Wasser, demin. | ad 100.0 |

**Beispiel 2**

| O/W-Creme | |
|---|---|
| Glycerylstearat | 0.50 |
| Tego Care CG 90 | 6.50 |
| Glycerin | 3.00 |
| Cetearylalkohol | 1.50 |
| Natriumhydroxid (45%ig) | 1.13 |
| Octyldodecanol | 7.0 |
| Capric/Caprylic Triglyceride | 5.0 |
| Cetearylisononanoat | 6.0 |
| Uvinul MS 40 | 3.0 |
| Carbomer | 0.2 |
| Konservierungsmittel | q.s. |
| Parfüm | q.s. |
| Wasser, demin. | ad 100.0 |

**Beispiel 3**

| O/W-Lotion | |
|---|---|
| Tego Care CG 90 | 5.50 |
| Butylenglykol | 5.00 |
| Cetearylalkohol | 0.50 |
| Xanthan Gum | 0.35 |
| $C_{12}$-$C_{15}$ Alkyl Benzoate | 10.0 |
| Eusolex 232 | 4.0 |
| NaOH (45%ig) | 1.3 |
| Cetearylisononanoat | 6.0 |
| Konservierungsmittel | q.s. |
| Parfum | q.s. |
| Wasser, demin. | ad 100.0 |

**Patentansprüche**

**1.** Kosmetische und dermatologische Emulsionen mit mindestens einer wäßrigen Phase, enthaltend

(a) eine wirksame Menge einer oder mehrerer grenzflächenaktiven Substanzen, gewählt aus der Gruppe der Alkylglucoside, welche sich durch die Strukturformel

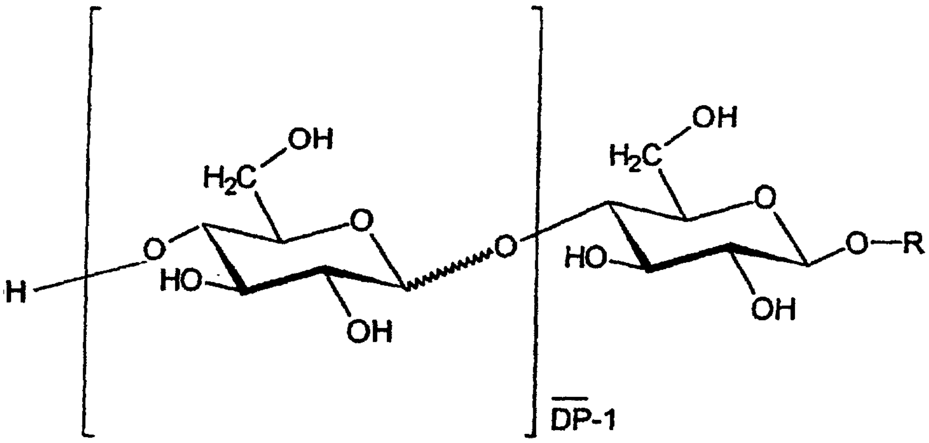

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet,
(b) wobei mindestens eine der wäßrigen Phasen einen oder mehrere Elektrolyte gelöst enthält, wobei die Ionenstärke der wäßrigen Phasen, in der der oder die Elektrolyte gelöst vorliegen, mindestens 0,075 mol/l beträgt.

**2.** Nichttherapeutische Verwendung von kosmetischen und dermatologischen Emulsionen, insbesondere O/W-Emulsionen mit mindestens einer wäßrigen Phase, enthaltend

(a) eine wirksame Menge einer oder mehrerer grenzflächenaktiven Substanzen, gewählt aus der Gruppe der Alkylglucoside, welche sich durch die Strukturformel

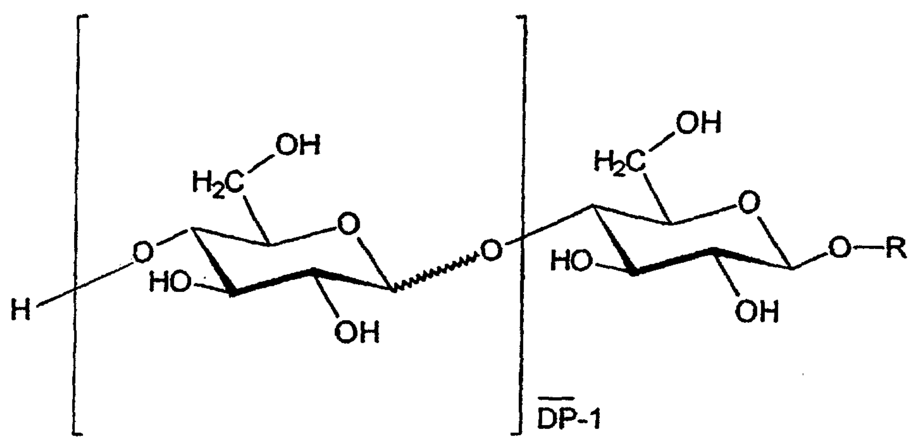

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet,
(b) wobei mindestens eine der wäßrigen Phasen einen oder mehrere Elektrolyte gelöst enthält, wobei die Ionenstärke der wäßrigen Phasen, in der der oder die Elektrolyte gelöst vorliegen, mindestens 0,075 mol/l beträgt,

zur Pflege der Haut.

**3.** Verwendung einer oder mehrerer grenzflächenaktiver Substanzen, gewählt aus der Gruppe der Alkylglucoside, welche sich durch die Strukturformel

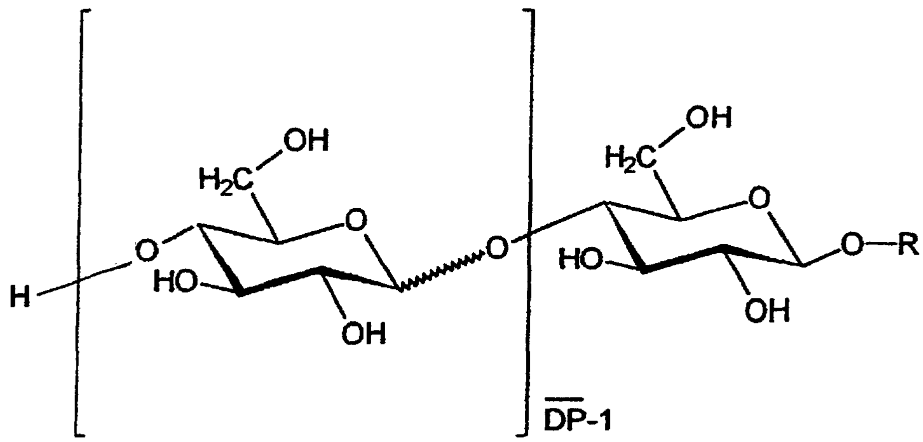

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet, zum Erzielen oder Erhöhen der Stabilität von Emulsionen gegenüber der Gegenwart von Elektrolyten, insbesondere zum Erzielen oder Erhöhen der Stabilität von O/W-Emulsionen gegenüber der Gegenwart von Elektrolyten.

**4.** Verwendung nach Anspruch 3 wobei die Emulsionen in mindestens einer der wäßrigen Phasen einen oder mehrere Elektrolyte gelöst enthält, und die lonenstärke der wäßrigen Phasen, in der der oder die Elektrolyte gelöst vorliegen, mindestens 0,075 mol/l beträgt.

**5.** Emulsionen nach Anspruch 1 oder Verwendung nach einer der Ansprüche 2 - 4, **dadurch gekennzeichnet, daß** $\overline{DP}$ gewählt wird aus dem Bereich von 1 - 2, bevorzugt von 1,1 - 1,5, insbesondere bevorzugt von ungefähr 1,3.

**6.** Emulsionen nach Anspruch 1 oder Verwendung nach einer der Ansprüche 2 - 4, **dadurch gekennzeichnet, daß** R gewählt wird aus der Gruppe der unverzweigten Alkylreste, wobei der Myristylrest, der Cetylrest, der Stearylrest und der Eicosylrest bevorzugt werden.

**7.** Emulsionen nach Anspruch 1 oder Verwendung nach einer der Ansprüche 2 - 4, **dadurch gekennzeichnet, daß** als grenzflächenaktive Substanzen, gewählt aus der Gruppe der Alkylglucoside, Gemische aus Stearylglucosid und Cetylglucosid verwendet werden.

**8.** Emulsionen nach Anspruch 1 oder Verwendung nach einer der Ansprüche 2 - 4, **dadurch gekennzeichnet, daß** die Gesamtmenge an einer oder mehreren erfindungsgemäß verwendeten grenzflächenaktiven Glucosederivaten in den fertigen kosmetischen oder dermatologischen Zubereitungen aus dem Bereich von 0,1 bis 25,0 Gew.-%, bevorzugt 0,5 bis 15,0 Gew.-% gewählt wird, bezogen auf das Gesamtgewicht der Zubereitungen.

**9.** Emulsionen nach Anspruch 1 oder Verwendung nach einer der Ansprüche 2 - 4, **dadurch gekennzeichnet, daß** der oder die Elektrolyte vorteilhaft gewählt aus der Gruppe

(1) der Salze mit folgenden Anionen: Chloride, femer anorganische Oxo-Element-Anionen, von diesen insbesondere Sulfate, Carbonate, Phosphate, Borate und Aluminate. Auch auf organischen Anionen basierende Elektrolyte sind vorteilhaft, z.B. Lactate, Acetate, Benzoate, Propionate, Tartrate, Citrate, Aminosäuren Ethylendiamintetraessigsäure und deren Salze
und folgenden Kationen: Ammonium,- Alkylammonium,- Alkalimetall-, Erdalkalimetall,- Magnesium-, Eisen- bzw. Zinkionen
(2) der zumeist als Alkalisalze vorliegende wasserlösliche UV-Filtersubstanzen, insbesondere solcher, die an ihrem Molekülgerüst eine oder mehrere Sulfonsäuregruppen bzw. Sulfonatgruppen tragen:
(3) der Aminosäuren und deren Salze bzw. deren Anionen
(4) der kosmetisch und dermatologisch relevanten $\alpha$-Hydroxycarbonsäuren, $\alpha$-Ketocarbonsäuren und $\beta$-Hydroxycarbonsäuren und insbesondere deren Salze, wobei die Kationen vorteilhaft gewählt werden können aus der Gruppe Ammonium,-Alkylammonium,- Alkalimetall-, Erdalkalimetall,- Magnesium-, Eisen- bzw. Zinkionen.

## Claims

1. Cosmetic and dermatological emulsions having at least one aqueous phase, comprising

(a) an effective amount of one or more interface-active substances, selected from the group consisting of alkyl glucosides, which are **characterized by** the structural formula

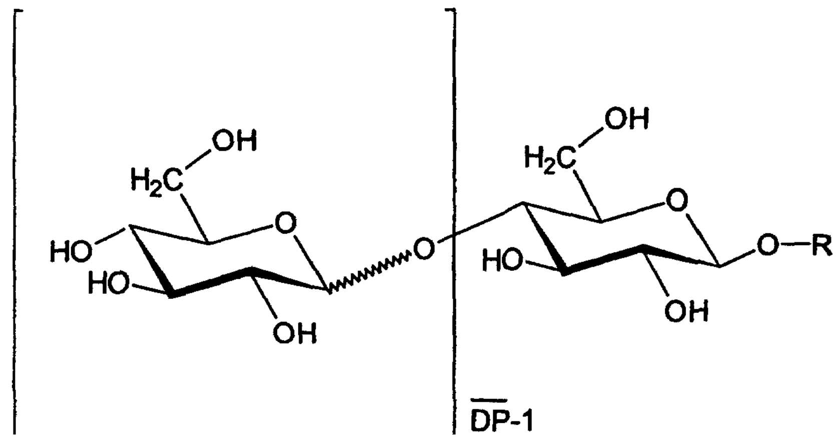

in which R is a branched or unbranched alkyl radical having from 4 to 24 carbon atoms and where $\overline{DP}$ is an average degree of glucosylation of up to 2,
(b) where at least one of the aqueous phases contains one or more electrolytes in dissolved form, the ionic strength of the aqueous phases in which the electrolyte(s) is/are present in dissolved form being at least 0.075 mol/l.

2. Non-therapeutic use of cosmetic and dermatological emulsions, in particular O/W emulsions having at least one aqueous phase, comprising

(a) an effective amount of one or more interface-active substances, selected from the group consisting of alkyl glucosides, which are **characterized by** the structural formula

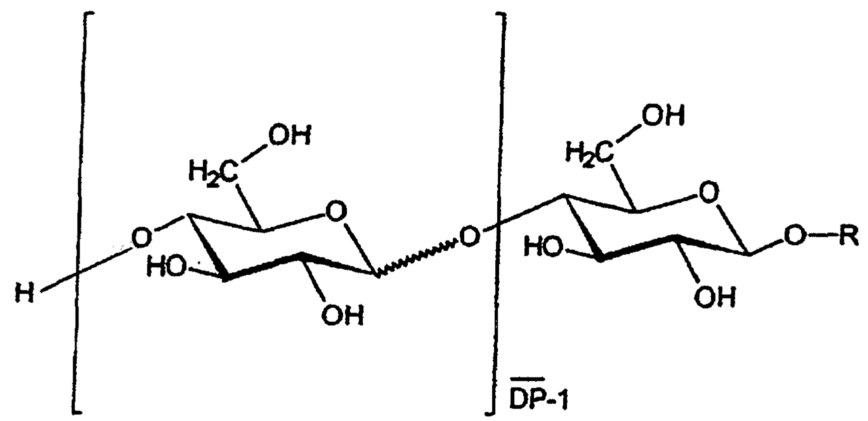

in which R is a branched or unbranched alkyl radical having from 4 to 24 carbon atoms and where $\overline{DP}$ is an average degree of glucosylation of up to 2,
(b) where at least one of the aqueous phases contains one or more electrolytes in dissolved form, the ionic strength of the aqueous phases in which the electrolyte(s) is/are present in dissolved form being at least 0.075 mol/l,

for skin care.

**3.** Use of one or more interface-active substances, selected from the group consisting of alkyl glucosides which are **characterized by** the structural formula

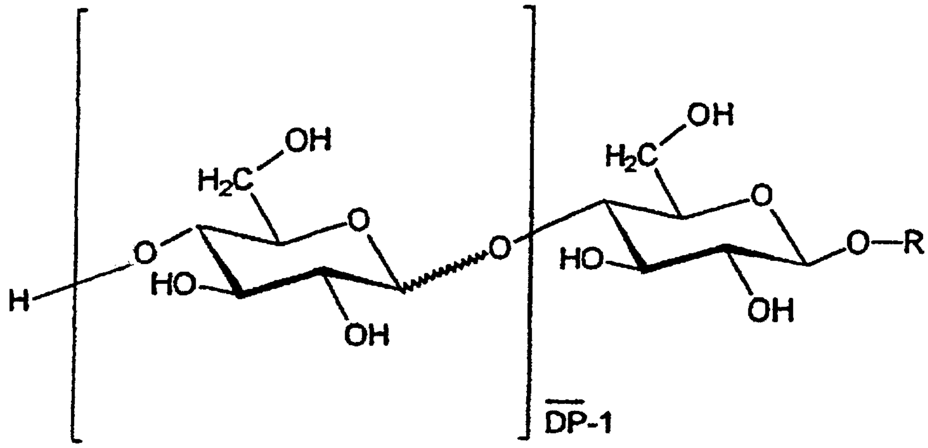

in which R is a branched or unbranched alkyl radical having from 4 to 24 carbon atoms, and where $\overline{DP}$ is an average degree of glucosylation of up to 2, for achieving or increasing the stability of emulsions to the presence of electrolytes, in particular for achieving or increasing the stability of O/W emulsions to the presence of electrolytes.

**4.** Use according to Claim 3, where the emulsions, in at least one of the aqueous phases, contains [sic] one or more electrolytes in dissolved form, and the ionic strength of the aqueous phases in which the electrolyte(s) is/are present in dissolved form is at least 0.075 mol/l.

**5.** Emulsions according to Claim 1 or use according to one of Claims 2 - 4, **characterized in that** $\overline{DP}$ is chosen from the range 1 - 2, preferably 1.1 - 1.5, particularly preferably about 1.3.

**6.** Emulsions according to Claim 1 or use according to one of Claims 2 - 4, **characterized in that** R is chosen from the group consisting of unbranched alkyl radicals, in which case the myristyl, cetyl, stearyl and eicosyl radicals are preferred.

**7.** Emulsions according to Claim 1 or use according to one of Claims 2 - 4, **characterized in that** the interface-active substances used are chosen from the group consisting of alkyl glucosides, mixtures of stearyl glucoside and cetyl glucoside.

**8.** Emulsions according to Claim 1 or use according to one of Claims 2 - 4, **characterized in that** the total amount of one or more interface-active glucose derivatives used according to the invention in the finished cosmetic or dermatological preparations is chosen from the range of from 0.1 to 25.0% by weight, preferably from 0.5 to 15.0% by weight, based on the total weight of the preparations.

**9.** Emulsions according to Claim 1 or use according to one of Claims 2 - 4, **characterized in that** the electrolyte(s) is/are advantageously chosen from the group consisting of

(1) salts containing the following anions: chlorides, also inorganic oxo-element anions, particularly sulfates, carbonates, phosphates, borates and aluminates. Electrolytes based on organic anions are also advantageous, e.g. lactates, acetates, benzoates, propionates, tartrates, citrates, amino acids, ethylenediaminetetraacetic acid and salts thereof
and the following cations: ammonium, alkylammonium, alkali metal, alkaline earth metal, magnesium, iron and zinc ions
(2) water-soluble UV filter substances which are mostly present as alkali metal salts, in particular those which carry on their molecular backbone one or more sulphonic acid groups or sulphonate groups:
(3) amino acids and salts thereof and anions thereof
(4) cosmetically and dermatologically relevant $\alpha$-hydroxycarboxylic acids, $\alpha$-ketocarboxylic acids and $\beta$-hydroxycarboxylic acids and, in particular, salts thereof, in which case the cations can advantageously be chosen from the group consisting of ammonium, alkylammonium, alkali metal, alkaline earth metal, magnesium, iron

and zinc ions.

## Revendications

1. Emulsions cosmétiques et dermatologiques avec au moins une phase aqueuse, contenant

   (a) une quantité efficace d'une ou de plusieurs substances tensioactives choisies dans le groupe des alkylglycosides, qui se caractérisent par la formule de structure

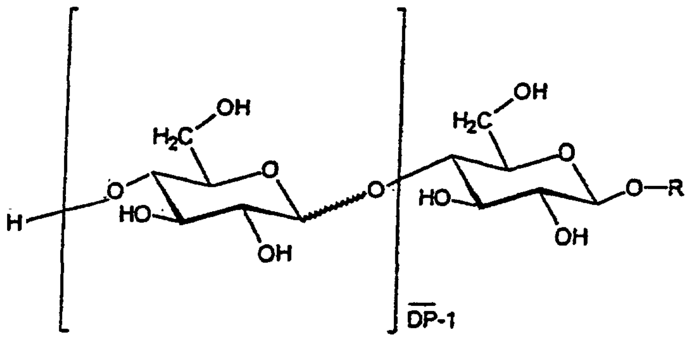

   R représentant un radical alkyle ramifié ou non ramifié comprenant 4 à 24 atomes de carbone et $\overline{DP}$ signifiant un degré moyen de glycosylation jusqu'à 2
   (b) au moins une des phases aqueuses contenant un ou plusieurs électrolytes dissous, la force ionique des phases aqueuses dans lesquelles le ou les électrolytes se trouvent à l'état dissous étant d'au moins 0,075 mole/l.

2. Utilisation non thérapeutique d'émulsions cosmétiques et dermatologiques pour les soins de la peau, en particulier des émulsions H/E avec au moins une phase aqueuse, contenant

   (a) une quantité efficace d'une ou de plusieurs substances tensioactives choisies dans le groupe des alkylglycosides, qui se caractérisent par la formule de structure

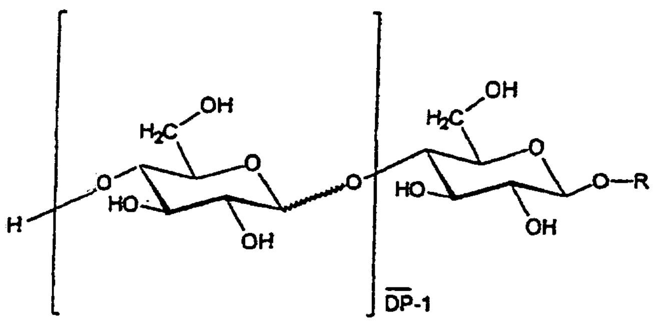

   R représentant un radical alkyle ramifié ou non ramifié comprenant 4 à 24 atomes de carbone et $\overline{DP}$ signifiant un degré moyen de glycosylation jusqu'à 2
   (b) au moins une des phases aqueuses contenant un ou plusieurs électrolytes dissous, la force ionique des phases aqueuses dans lesquelles le ou les électrolytes se trouvent à l'état dissous étant d'au moins 0,075 mole/l.

3. Utilisation d'une ou de plusieurs substances tensioactives, choisies dans le groupe des alkylglycosides, qui se caractérisent par la formule de structure

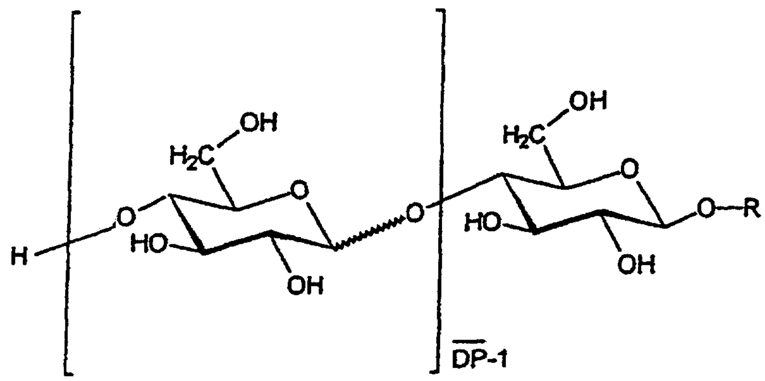

R représentant un radical alkyle ramifié ou non ramifié comprenant 4 à 24 atomes de carbone et $\overline{DP}$ signifiant un degré moyen de glycosylation jusqu'à 2 pour obtenir ou augmenter la stabilité d'émulsions par rapport à la présence d'électrolytes, en particulier pour obtenir ou augmenter la stabilité d'émulsions H/E par rapport à la présence d'électrolytes.

**4.** Utilisation selon la revendication 3, les émulsions contenant dans au moins une des phases aqueuses un ou plusieurs électrolytes dissous et la force ionique des phases aqueuses dans lesquelles le ou les électrolytes se trouvent à l'état dissous étant d'au moins 0,075 mole/l.

**5.** Emulsions selon la revendication 1 ou utilisation selon l'une quelconque des revendications 2 à 4, **caractérisées en ce que** $\overline{DP}$ est choisi dans la plage de 1 à 2, de préférence de 1,1 à 1,5, de manière particulièrement préférée d'environ 1,3.

**6.** Emulsions selon la revendication 1 ou utilisation selon l'une quelconque des revendications 2 à 4, **caractérisées en ce que** R est choisi dans le groupe des radicaux alkyle non ramifiés, les radicaux myristyle, cétyle, stéaryle et eicosyle étant préférés.

**7.** Emulsions selon la revendication 1 ou utilisation selon l'une quelconque des revendications 2 à 4, **caractérisées en ce qu'**on utilise comme substances tensioactives, choisies dans le groupe des alkylglycosides, des mélanges de glycoside de stéaryle et de glycoside de cétyle.

**8.** Emulsions selon la revendication 1 ou utilisation selon l'une quelconque des revendications 2 à 4, **caractérisées en ce que** la quantité totale d'un ou de plusieurs dérivés de glucose tensioactifs utilisés selon l'invention dans les préparations cosmétiques ou dermatologiques finies est choisie avantageusement dans la plage de 0,1 à 25,0% en poids, de préférence de 0,5 à 15,0% en poids, par rapport au poids total des préparations.

**9.** Emulsions selon la revendication 1 ou utilisation selon l'une quelconque des revendications 2 à 4, **caractérisées en ce que** le ou les électrolytes sont avantageusement choisis dans le groupe

(1) des sels avec les anions suivants : chlorures, de plus anions inorganiques d'éléments oxo, parmi ceux-ci en particulier les sulfates, carbonates, phosphates, borates et aluminates. Des électrolytes à base d'anions organiques sont également avantageux, par exemple les lactates, acétates, benzoates, propionates, tartrates, citrates, acides aminés, acide éthylènediaminetétraacétique et ses sels et les cations suivants : ions d'ammonium, d'alkylammonium, de métal alcalin, de métal alcalino-terreux, de magnésium, de fer ou, selon le cas, de zinc
(2) des substances filtre des UV solubles dans l'eau, se trouvant le plus souvent sous forme de sels alcalins, en particulier celles qui portent sur leur structure moléculaire un ou plusieurs groupements acide sulfonique ou, selon le cas, sulfonate;
(3) des acides aminés et leurs sels ou, selon le cas, leurs anions
(4) des acides α-hydroxycarboxyliques, α-cétocarboxyliques et β-hydroxycarboxyliques cosmétiquement et dermatologiquement importants et en particulier leurs sels, les cations pouvant être choisis avantageusement dans le groupe des ions d'ammonium, d'alkylammonium, de métal alcalin, de métal alcalino-terreux, de magnésium, de fer ou, selon le cas, de zinc

Fig. 1